# EUROPÄISCHE PATENTANMELDUNG

(11) **EP 2 503 478 A2**
(43) Veröffentlichungstag der Anmeldung: **26.09.2012**
(21) Anmeldenummer: 11159224.2
(22) Anmeldetag: 22.03.2011
(51) Int. Cl.: G06F 19/00

(54) **Vorrichtung und Verfahren zur sicheren Signaleingabe**

(71) Anmelder: ERBE Elektromedizin GmbH, 72072 Tübingen (DE)
(72) Erfinder: Tomaschko, Stefan, 72411 Bodelshausen (DE); Notz, Jürgen, 72581 Dettingen (DE)
(74) Vertreter: Rüger, Barthelt & Abel

(57) **Zusammenfassung**

Ein medizinisches Gerät (10) ist mit einer vorzugsweise berührungsempfindlichen Eingabeeinrichtung (15) versehen, die durch eine Sperreinrichtung (25) in eine inaktive Betriebsart versetzt werden kann, in der sie gegen Störimpulse weniger anfällig ist oder in der von ihr empfangene Signale nicht an einen Verarbeitungsblock (26) weitergegeben werden. Durch Betätigung einer Entriegelungstaste, durch Vornahme einer bestimmten Entriegelungsgeste unter Berührung der Eingabeeinrichtung (15) oder durch längere Berührung derselben, kann die Eingabeeinrichtung (15) wieder für Eingaben reaktiviert werden. Alternativ kann die Aktivierung durch anderweitige Sensoren erfolgen, die die tatsächliche Berührung der Eingabeeinrichtung (15) anhand gesonderten physikalischer Größen, wie Berührungskraft oder auch optisch erfassen. Die Signalfreigabe erfolgt hier nur bei Zusammentreffen der berührungssensitiv erfassten Signale des Eingabeelements (15) mit den Signalen der anderweitigen Erfassungseinrichtung. Fehleingaben werden so vermieden.

## Beschreibung

Die Erfindung betrifft ein medizinisches Gerät mit geschützten Bedienelementen und ein Verfahren zur sicheren Befehlseingabe.

Medizinische Geräte, wie beispielsweise Geräte zur Endoskopie, HF-chirurgische Geräte und anderweitige medizinische Geräte haben meist eine Vielzahl von Bedienelementen und mindestens ein Ausgabeelement, beispielsweise einen Bildschirm. Der Bildschirm kann beispielsweise als Touchscreen ausgebildet sein, wie er beispielsweise aus der DE 11 2008 000 906 T5 bekannt ist. Der Touchscreen weist eine relativ große Eingabefläche auf, die mit Elektroden überzogen ist, um lokale Berührungen kapazitiv zu erfassen. Die großflächigen oder über einen weiten Bereich gespannten Elektroden können wie Antennen Störsignale auffangen und deshalb Eingaben empfangen, die keinen Nutzereingaben entsprechen.

Ein weiterer berührungsempfindlicher Bildschirm ist aus der EP 1 496 425 B1 bekannt. Dieser nutzt die durch Berührung hervorgerufene lokale Verformung und eine sich daraus ergebende Kontaktgabe. Auch dieser Touchscreen benötigt großflächige oder über weite Bereiche gespannte Elektroden, die Störsignale und somit scheinbare Eingeben auffangen können.

Weitere Eingabemittel, wie Schalter, insbesondere Sensorschalter, Potentiometer oder sonstige Eingabemittel, können ebenfalls Störsignale auffangen und zwar insbesondere dann, wenn sie über längere Leitungen mit einer Auswerteeinheit verbunden sind. Dies betrifft insbesondere Schalter, die nicht direkt an dem elektromedizinischen Gerät sondern z.B. an einem über ein langes Kabel mit dem Gerät verbundenen Instrument vorhanden sind.

Im Operationssaal tritt insbesondere in unmittelbarer Nachbarschaft HF-chirurgischer Geräte ein hoher elektromagnetischer Störpegel auf, der insbesondere an Touchscreens, aber auch an anderen Eingabemitteln zu scheinbaren Betätigungssignalen und somit zu Fehleingaben führen kann. Dies kann eine erhebliche nicht zu vernachlässigende Gefahrenquelle darstellen.

Es ist deshalb Aufgabe der Erfindung, ein Konzept anzugeben, mit dem sich solche fehlerhaften, auf Störungen beruhenden Eingaben vermeiden lassen.

Diese Aufgabe wird mit dem Gerät nach Anspruch 1, wie auch mit dem Verfahren nach Anspruch 11 oder alternativ Anspruch 12 gelöst:
Das erfindungsgemäße Gerät weist eine Steuereinrichtung auf, die Baugruppe steuert, die an einem Patienten eine Wirkung erbringt oder anderweitig mit diesem zusammenwirkt. Eine solche Baugruppe kann beispielsweise ein chirurgisches Instrument, wie z.B. ein elektrisches Skalpell, eine Koagulationszange, eine Sonde, ein kryochirurgisches Instrument, ein wasserstrahlschneidendes Instrument, ein Endoskop, ein anderweitiges Endgerät oder auch eine Beatmungsvorrichtung, ein Messinstrument oder dergleichen sein. Zur Steuerung dieser Baugruppe benötigt die Steuereinrichtung häufig Nutzereingaben, die über eine entsprechende Eingabeeinrichtung gegeben werden. Die Eingabeeinrichtung kann beispielsweise eine oder mehrere berührungssensitive Flächen umfassen und somit beispielsweise als Touchscreen ausgebildet sein. Die bei bevorzugten Ausführungsformen verwendeten Touchscreens haben den Vorzug, viele Eingabemöglichkeiten geordnet und übersichtlich darbieten zu können, so dass eine intuitive Bedienung möglich ist und durch Irrtum oder Unkenntnis erfolgende Fehleingaben weitestgehend vermieden werden. Sie tragen somit zur Sicherheit im Operationssaal bei. Andererseits sind Eingabeelemente generell und Touchscreens im Besonderen empfindlich gegen elektromagnetische Störfelder.

Das erfindungsgemäße Gerät weist ein Sperrmodul auf, das solche empfindlichen Eingabeelemente wenigstens teilweise und wenigstens zeitweilig gegen Eingaben sperrt. Damit können HF-Störimpulse, die ansonsten zu zufälligen und somit fehlerhaften Eingaben führen könnten, sicher ausgeblendet werden. Es können an einem Patienten HF-chirurgische Eingriffe vorgenommen werden, ohne dass in der Nähe stehende erfindungsgemäß ausgebildete Geräte dadurch unerwünschte Eingaben empfangen und somit fehlerhafte Funktionen erbringen. Die fehlerhafte Verstellung von Parametern, Aktivierung oder Deaktivierung von Geräten und ähnliche Fehlerzustände werden somit sicher ausgeschlossen.

Das Sperrmodul kann seine Sperrwirkung auf unterschiedliche Weise erbringen. Es kann beispielsweise so ausgebildet sein, dass es die von dem Eingabeelement gelieferten Signale auf eine bestimmte Signatur überprüft und die Signale nur dann zur weiteren Verarbeitung frei gibt, wenn die bestimmte Signatur erkannt worden ist. Diese bestimmte Signatur, die eine bestimmte, charakteristische Nutzereingabe kennzeichnet, kann bei einem Touchscreen beispielsweise darin bestehen, dass ein bestimmtes Feld über eine bestimmte Mindestzeitspanne berührt worden ist. Die Mindestzeitspanne kann, je nach Anwendungsfall, von wenigen zehntel Sekunden bis zu mehreren Sekunden gewählt werden. Es ist auch möglich, die Signatur zu nutzen, die eine bestimmte Eingabegeste erbringt, wie z.B. beispielsweise eine linienhafte, drehenden oder sonstigen Wischbewegung. Es können auch Zeitfolgesignaturen abgefragt werden. Beispielsweise kann zur Aktivierung eines sonst gesperrten Touchscreens ein bestimmtes Feld in bestimmter zeitlicher Folge mehrmals hintereinander oder es können bestimmte ausgewählte Felder abwechselnd berührt werden. Die Wahrscheinlichkeit, dass eine zufällige HF-Störung genau diese Signatur hat, kann je nach Kompliziertheit der Signatur auf praktisch Null reduziert werden.

Es ist weiter möglich, das Sperrmodul so auszubilden, dass die Sensorik eines Eingabeelements, d.h. beispielsweise die Sensorelektroden eines Touchscreens zur Sperrung desselben völlig deaktiviert werden, also keine Abfragesignale erhalten und/oder nicht ausgelesen werden.

Es ist möglich, das Sperrmodul so auszubilden, dass es nach einer vorgegebenen Zeit nicht erkannter Bedienungen von sich aus in einen Sperrmodus übergeht, aus dem es nur durch mindestens eine der oben genannten Maßnahmen wieder entsperrt werden kann. Es ist auch möglich, das Sperrmodul so auszubilden, dass es durch Betätigung eines Felds auf dem Touchscreen oder eines Sperrknopfs an dem Gerät willkürlich gesperrt und/oder entsperrt werden kann.

Es ist weiter möglich, den Touchscreen oder das sonstige Eingabeelement mit einer ergänzenden Sensoreinrichtung zusammenwirkend zu verbinden, mit deren Hilfe eine tatsächliche willkürliche Betätigung von einer stochastischen HF-Störung zu unterscheiden ist. Dazu kann beispielsweise eine Krafterfassungseinrichtung dienen, die eine Berührung des Touchscreens oder sonstigen Eingabeelements durch einen Bediener anhand der ausgeübten Kraft erkennt. In einer verfeinerten Ausführungsform können dazu mehrere Kraftsensoren, z.B. an den Ecken eines Touchscreendisplays vorgesehen sein, wobei sich aus der Größe der verschiedenen erfassten Kräfte der Ort der Berührung wenigstens grob lokalisieren lässt. Eine gültige Eingabe kann die Steuereinrichtung z.B. daran erkennen, dass der durch die Kräfte erfasste Ort wenigstens näherungsweise mit dem von dem Touchscreen erfassten Berührungsort übereinstimmt. Ist dies nicht der Fall, kann das Sperrmodul die jeweilige Eingabe sperren.

Ähnlich kann mit einem optischen Hilfssensor vorgegangen werden, der zusätzlich zur über den Touchscreen erfassten Eingabe erkennt, ob der Touchscreen tatsächlich berührt worden ist oder nicht. Entsprechend können Eingabesignale wieder als gültig freigegeben oder als ungültig gesperrt werden.

Das Sperrmodul kann eine gesonderte Baugruppe sein. Diese Baugruppe kann in den Touchscreen oder das sonstige Eingabeelement integriert sein. Alternativ kann sie separat zwischen Eingabeelement und Steuereinrichtung angeordnet sein. Wiederum alternativ kann das Sperrmodul auch in die Steuereinrichtung integriert sein. Das Sperrmodul kann sowohl hardwaretechnisch, als auch als Softwaremodul ausgebildet sein.

Nach Anspruch 11 kann das Sperrmodul Eingabesignale von störungsverursachten Scheineingabesignalen unterscheiden, indem nur solche Eingabesignale als gültig zugelassen werden, die in vorgegebener Koinzidenz mit anderen Eingabesignalen stehen. Diese anderen Eingabesignale kennzeichnen wohldefinierte Ereignisse, deren stochastisches Zustandekommen äußerst unwahrscheinlich ist. Solche anderen Ereignisse können das Betätigen einer Entriegelungstaste sein. Weiter kann ein solches anderes Eingabesignal, z.B. eine mit dem Touchscreen erfasste Schiebebewegung oder auch längeres Betätigen eines bestimmten Tastenfelds sein. Die Koinzidenz kann auf einen bestimmten Zeitraum von z.B. mehreren, z.B. 15 Sekunden beschränkt sein. Nach Ablauf dieser Zeit ohne Erfassung einer weiteren Eingabe kann das Sperrmodul wieder in den Sperrmodus übergehen und alle weiteren Eingaben sperren.

Alternativ kann das Verfahren nach Anspruch 12 vorsehen, dass die Eingabeeinrichtung außerhalb ihres aktivierten Zustands völlig inaktiviert ist oder z.B. in einen Halbschlafmodus übergeht. Bei einem kapazitiven Touchscreen kann dieser Halbschlafmodus z.B. darin bestehen, dass seine Abtastrate stark verlangsamt ist. Elektromagnetische Störungen, die in diesem Zustand auf den Touchscreen treffen, müssten über längere Zeit mit exakt gleicher Charakteristik anliegen, um einen Tastendruck, also eine gewünschte Betätigung, vorzutäuschen, was extrem unwahrscheinlich ist. Damit ist auch diese Lösung eine sehr wirksame risikomindernde Maßnahme.

Weitere Einzelheiten vorteilhafter Ausführungsformen der Erfindung sind Gegenstand von Ansprüchen, der Zeichnung oder der Beschreibung.

Es zeigen:
Fig. 1 ein medizinisches Gerät mit Ein- und Ausgabeelementen, in schematisierter Darstellung,
Fig. 2 bis 4 Ausführungsformen der Gerätesteuerung, in schematisierter Blockdarstellung,
Fig. 5 eine Eingabeeinrichtung des Geräts nach Fig. 1 mit Touchscreen und optischer Berührungsüberwachung, als schematisiertes Blockbild, und
Fig. 6 eine Eingabeeinrichtung mit Touchscreen und zusätzlicher Kräfteüberwachung, als schematisiertes Blockbild.

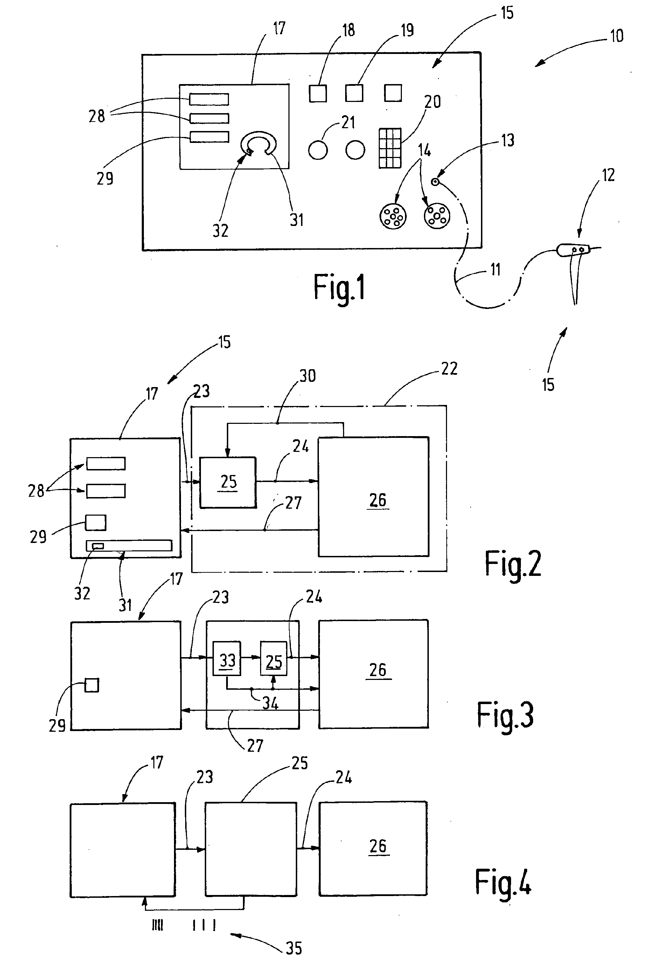

In Fig. 1 ist ein medizinisches Gerät 10 veranschaulicht, das z.B. als elektrochirurgisches Gerät zum Betrieb eines über eine längere Leitung 11 angeschlossenen chirurgischen Instruments 12, einer Messsonde oder dergleichen ausgebildet sein kann. Es kann sich bei dem Gerät 10 jedoch auch um ein anderes medizinisches Gerät, wie beispielsweise ein Beatmungsgerät, ein zum Betrieb eines Endoskops eingerichtetes Gerät oder ein sonstiges medizinisches Gerät handeln. Insoweit dient das Instrument 12 hier nur zur Veranschaulichung irgendeiner Baugruppe, die an das Gerät 10 angeschlossen oder auch Teil desselben sein kann. Im vorliegenden Fall ist das Instrument 12 über die Leitung 11 an eine entsprechende Buchse 13 angeschlossen. Weitere Buchsen 14 zum Anschluss ähnlicher oder anderer Instrumente können vorgesehen sein.

An dem Instrument 12, wie auch an dem Gerät 10, können Bedien- bzw. Eingabeelemente 15 vorgesehen sein. Die Eingabeelemente 15 des Instruments 12 können beispielsweise Taster 16 sein. Die Eingabeelemente 15 des Geräts 10 können ein Touchscreen 17, berührungssensitive oder auch druckempfindliche Tasten 18, 19, Bedienfelder 20, resistive oder optische Potentiometer 21 und dergleichen sein. Der Touchscreen 17 kann zugleich als Anzeigeeinheit dienen, an der Messwerte, Einstellwerte, Berechnungsresultate und dergleichen dargestellt werden. Ebenso können die Drucktasten 18, 19 oder das Bedienfeld 20 als Anzeigeelemente dienen, indem beispielsweise Hinterleuchtungen in Helligkeit, Farbe, Leuchtfrequenz oder dergleichen geändert werden können, um Signale zu geben. Gleiches gilt für die Potentiometer 21.

Zumindest einige der Eingabeelemente 15 können prinzipiell empfindlich gegen elektromagnetische Störungen oder Einstrahlungen sein. Dies gilt beispielsweise für den Touchscreen 17. Die Tendenz zu möglichst großen Anzeige-und Wiedergabeflächen führt hier zu großflächigen Elektroden, die als Antennen wirken und elektromagnetische Störungen empfangen können. Störungen können auch in das Bedienfeld 20 einstrahlen, das ebenfalls großflächige Elektroden haben kann. Auch kann die Leitung 11 Störsignale auffangen und schlimmstenfalls manuelle Betätigungen der Taster 16 auslösen.

Nachfolgend wird insbesondere beschrieben, wie der Touchscreen 17 gegen derartige Störungen unempfindlich gemacht werden kann. Diese Prinzipien können ohne weiteres auch auf alle übrigen Bedienelemente 15 angewandt werden.

Fig. 2 veranschaulicht den Touchscreen 17 und eine an diesen angeschlossene Steuereinrichtung 22 als Blockschaltbild. Der Touchscreen 17 liefert Signale 23 an die Steuereinrichtung 22, wobei die Signale 23 sowohl Scheineingabesignale als auch echte Eingabesignale 24 sein können. Die Steuereinrichtung 22 enthält ein Sperrmodul 25, das die Weitergabe von Scheineingabesignalen an einen weiteren Verarbeitungsblock 26 unter bestimmten Bedingungen sperrt.

Das Sperrmodul 25 und der Verarbeitungsblock 26 können als Teil der Steuereinrichtung 22 z.B. in Form von Software ausgebildet sein, die auf einem geeigneten Prozessor läuft. Dabei werden z.B. folgende Abläufe realisiert:
Der Verarbeitungsblock 26 gibt Wiedergabesignale 27 an den Touchscreen 17. Dadurch erscheinen auf dem Touchscreen 17 entsprechende Repräsentationen 28, beispielsweise in Gestalt von Eingabemasken, berührungsaktiven Tastenfeldern 29 oder dergleichen. Der Touchscreen 17 kann dann beispielsweise für eine kurze Zeit (z.B. 15 Sekunden) auf Eingaben warten. Dies erfolgt, indem der Verarbeitungsblock 26 dem Sperrmodul 25 die Bereitschaft signalisiert, Daten aufzunehmen. Das Sperrmodul 25 gibt somit den Weg für Daten von dem Touchscreen 17 zu dem Verarbeitungsblock 26 frei. Die Signale 23 werden als Eingabesignale 24 an den Verarbeitungsblock 26 gegeben.

Z.B. nach einer festgelegten Zeitspanne kann das System in einen Verriegelungsmodus übergehen. Der Verarbeitungsblock 26 gibt dazu ein Sperrsignal 30 an das Sperrmodul 25. Dieses unterbindet dann die Weitergabe von Signalen 23 an den Verarbeitungsblock 26. Zugleich oder in engem zeitlichen Zusammenhang kann der Verarbeitungsblock 26 entsprechende Wiedergabesignale 27 an den Touchscreen 17 senden, um dort beispielsweise den verriegelten Zustand anzuzeigen und Entriegelungsmittel anzugeben. Die Verriegelung der Eingabe kann in einer Abdunklung des Touchscreens 17, in einer Farbänderung desselben, in der Wiedergabe eines entsprechenden Anzeigefelds oder dergleichen bestehen. Zugleich kann auf dem Touchscreen 17 ein spezielles Feld 31 erscheinen, das zum Entsperren des Sperrmoduls 25 genutzt werden kann. Dieses spezielle Feld 31 kann beispielsweise einen bildlich dargestellten Schieber 32 enthalten, der zur Entsperrung der Eingabe zügig innerhalb des Felds 31 in vorgegebener Richtung verschoben werden muss. Dadurch entstehen Signale 23 mit einer bestimmten Signatur, die das Verschieben des Schiebers 32 anzeigt. Das Sperrmodul 25 kann einen Diskriminatorblock enthalten, der diese Signatur erkennt und das Sperrmodul 25 daraufhin entsperrt. Somit werden weitere Eingaben, die Signale 23 erzeugen, als Eingabesignale 24 an den Verarbeitungsblock 26 weitergegeben. Nach erfolgter Eingabe, wenn über längere Zeit, z.B. mehrere, z.B. 15 Sekunden keine weiteren Signale empfangen werden, kann der Verarbeitungsblock 26 das Sperrmodul 25 wieder sperren.

In gesperrtem Zustand können von dem Touchscreen 17 aufgefangene Störimpulse möglicherweise zwar Signale 23 erzeugen. Jedoch werden diese nicht an den Verarbeitungsblock 26 weitergegeben. Der Touchscreen 17 ist somit unempfindlich gegen Störungen. Fehleingaben können nicht erfolgen.

Es sind Abwandlungen möglich. Anstelle einer linearen Schiebebewegung des Schiebers 32 als entsperrende Nutzereingabe können andere Gesten zur Entsperrung des Sperrmoduls 25 genutzt werden, wie beispielsweise eine kreisförmige oder kreisbogenförmige Geste mit dem Schieber 32, wenn das Feld 31 entsprechend geformt ist (Fig. 1).

Es ist auch möglich, sonstige Zeichen zur Entsperrung des Touchscreens 17 anzuwenden. Z.B. kann vorgesehen sein, zur Entsperrung das berührungsaktive Tastenfeld 29 oder eine andere Taste oder ein anderes Feld längere Zeit, z.B. mindestens eine Sekunde zu berühren. Eine solche Ausführungsform ist in Fig. 3 veranschaulicht. Zwischen dem Sperrmodul 25 und dem Touchscreen 17 ist ein Diskriminatormodul 33 angeordnet. Dieses kann durch Hardware, Software und gegebenenfalls auch als Bestandteil des Sperrmoduls 25 und/oder des Touchscreens 17 realisiert sein. Das Diskriminatormodul 33 kann die Signale 23 auf Vorliegen einer bestimmten Signatur untersuchen, die sich beispielsweise ergibt, wenn ein bestimmtes Feld, z.B. das Tastenfeld 29, längere Zeit betätigt wird und danach das Sperrmodul 25 freischalten. Die Freischaltung kann bestehen bleiben, bis die Eingabe beendet und womöglich eine Zusatzzeitspanne abgelaufen ist. Der Touchscreen 17 wird danach wieder gesperrt. Der Verarbeitungsblock 26 erhält das entsprechende Sperrsignal 34 und zeigt die Sperrung auf dem Touchscreen 17 über ein entsprechendes Wiedergabesignal 27 an.

Anstelle der Signalsignatur, die durch eine längere Berührung des Tastenfelds 29 erhalten wird, kann jede andere Signatur ausgewertet werden, wie beispielsweise die auf bestimmte Eingaben zurückgehende Signatur oder dergleichen. Solche Eingaben können auch Codeeingaben, Eingaben von Kennnummern, Kennwörtern, ähnlich Passwörter, oder dergleichen sein.

Der Touchsceen kann in eingabegesperrtem Zustand weiterhin als Anzeigebildschirm dienen und Messwerte, Messkurven oder sonstige Anzeigen erbringen.

Bei den vorstehend beschriebenen Ausführungsbeispielen ist der Touchscreen 17 im gesperrten Zustand selbst unverändert aktiv geblieben. Die Störungsunterdrückung erfolgte durch Sperrung der Signalübertragung mittels des Sperrmoduls 25 gemäß Figur 2 oder 3. Es ist aber auch möglich, den Touchscreen 17 selbst in eine weniger störungsempfindliche Betriebsart zu überführen (Figur 4). Das Sperrmodul 25 ist hierzu funktionsmäßig eng mit dem Touchscreen 17 verzahnt. Es liefert oder beeinflusst die Erzeugung von Abfrageimpulsen 35 mit denen die einzelnen Elektroden des Touchscreens 17 auf Berührung des Touchscreen gescannt werden. Normalerweise wird der Touchscreen 17 im Aktivmodus mit hoher Frequenz abgefragt. In diesem Modus können normale Eingaben durch leichtes Berühren entsprechender Felder gemacht werden. Erfolgt längere Zeit keine Eingabe, geht das Sperrmodul 25 in Sperrmodus über. Schnelle Eingaben sind nun nicht mehr möglich. Der Touchscreen 17 wird nun in einem Halbschlafmodus betrieben (Slow Modus), indem die Abfrageimpulse 35 mit deutlicher verminderter Frequenz aufeinander folgen. In diesem Zustand ist der Touchscreen 17 wenig störempfindlich. Kurze Störimpulse werden in diesem Halbschlafmodus nicht in Signale 23 umgesetzt. Setzt ein Bediener jedoch seinen Finger längere Zeit auf den Touchscreen 17 ergibt dies ein Signal 23, welches das Sperrmodul 25 entsperrt. Es wird nun zur schnellen Abfrage des Touchscreens 17 zurückgekehrt und die Signale 23 werden an den Verarbeitungsblock 26 gegeben.

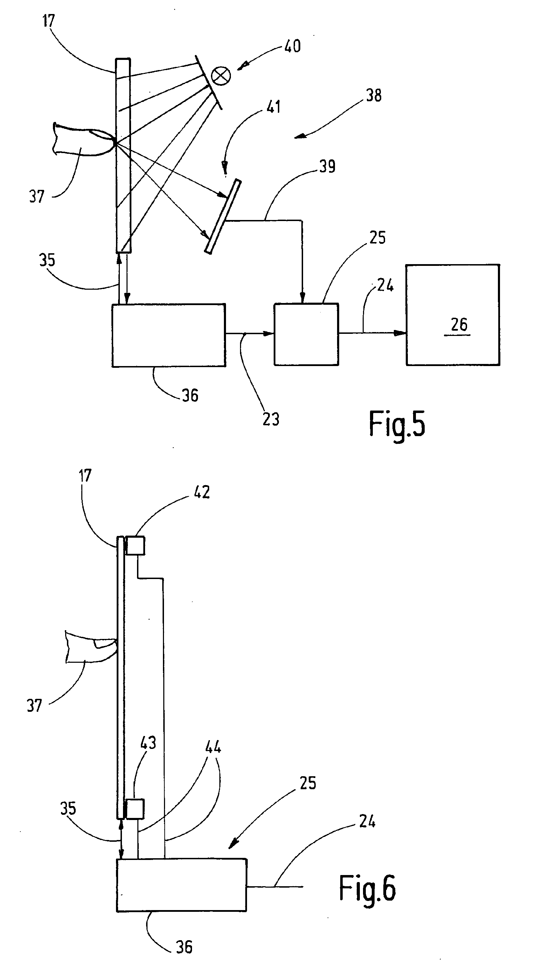

Fig. 5 veranschaulicht eine weitere Ausführungsform des erfindungsgemäßen Systems. Zu dem Touchscreen 17 gehört eine Treiberelektronik 36, die aus den Berührungen z.B. eines Fingers 37 die Signale 23 generiert. Zusätzlich kann über ein optisches Erfassungssystem 38 ein Signal 39 erzeugt werden, das die Berührung des Touchscreens 17 durch den Finger 37 erfasst. Zum Beispiel kann eine hinter dem Touchscreen 17 angebrachte Beleuchtung 40 in Verbindung mit einem Reflexlichtsensor 41 dazu dienen, die Berührung durch den Finger 37 zu erfassen. Das Sperrmodul 25 lässt Signale 23 nur als Eingabesignal 24 an den Verarbeitungsblock 26 durch, wenn es zugleich ein entsprechendes Freigabesignal 39 von dem optischen Erfassungssystem 38 empfängt.

Eine abgewandelte Ausführungsform zeigt Figur 6. Dort wird die von einem Finger 37 auf den Touchscreen 17 ausgeübte Kraft mittels mindestens eines, vorzugsweise mehrere Kraftsensoren 42, 43 erfasst, die zum Beispiel an den Ecken des Touchscreens 17 oder auch an andere geeigneten Orten angebracht sind. Die Treiberelektronik 36 bedient hier einerseits den Touchscreen 17 und wertet andererseits zusätzlich die von den Kraftsensoren 42, 43 gelieferten Signale 44 aus. Im einfachsten Fall wird nur auf ein gültiges Touchscreensignal geschlossen, wenn zugleich Signale der Kraftsensoren 42, 43 vorliegen.

Bei einer verfeinerten Ausführungsform kann die Treiberelektronik 36 aus der Größe der Signale der Kraftsensoren 42, 43 wenigstens grob den Ort der Berührung durch den Finger 37 bestimmen. Stimmt dieser Ort mit dem von dem Touchscreen 17 erfassten Ort überein und stimmt außerdem die erfasste Berührungsdauer überein, wird ein entsprechendes Eingabesignal 24 ab- und weitergegeben. Wird keine Übereinstimmung festgestellt, wird das betreffende Signal unterdrückt. Die Treiberelektronik 36 übernimmt somit die Funktion des Sperrmoduls 25.

Ein medizinisches Gerät 10 ist mit einer vorzugsweise berührungsempfindlichen Eingabeeinrichtung 15 versehen, die durch eine Sperreinrichtung 25 in eine inaktive Betriebsart versetzt werden kann, in der sie gegen Störimpulse weniger anfällig ist oder in der von ihr empfangene Signale nicht an einen Verarbeitungsblock 26 weitergegeben werden. Durch Betätigung einer Entriegelungstaste, durch Vornahme einer bestimmten Entriegelungsgeste unter Berührung der Eingabeeinrichtung 15 oder durch längere Berührung derselben, kann die Eingabeeinrichtung 15 wieder für Eingaben reaktiviert werden. Alternativ kann die Aktivierung durch anderweitige Sensoren erfolgen, die die tatsächliche Berührung der Eingabeeinrichtung 15 anhand gesonderter physikalischer Größen, wie Berührungskraft oder auch optisch erfassen. Die Signalfreigabe erfolgt hier nur bei Zusammentreffen der berührungssensitiv erfassten Signale des Eingabeelements 15 mit den Signalen der anderweitigen Erfassungseinrichtung. Fehleingaben werden so vermieden.

### Bezugszeichenliste:

- 10: Gerät
- 11: Leitung
- 12: Instrument
- 13: Buchse für Instrument 12
- 14: weitere Buchsen
- 15: Eingabeelemente, Eingabeeinrichtung
- 16: Taster
- 17: Touchscreen
- 18, 19: Drucktasten
- 20: Bedienfeld
- 21: Potentiometer
- 22: Steuereinrichtung
- 23: Signal
- 24: Eingabesignal
- 25: Sperrmodul, Sperreinrichtung
- 26: Verarbeitungsblock
- 27: Wiedergabesignale
- 28: Eingabemaske auf dem Touchscreen 17
- 29: Tastenfeld auf dem Touchscreen 17
- 30: Sperrsignal
- 31: Feld
- 32: Schieber
- 33: Diskriminatormodul
- 34: Sperrimpuls, Sperrsignal
- 35: Abfrageimpuls
- 36: Treiberelektronik, Vergleichseinrichtung
- 37: Finger
- 38: optisches Erfassungssystem
- 39: Signal
- 40: Beleuchtung
- 41: Reflexlichtsensor
- 42, 43: Kraftsensor, Krafterfassungseinrichtung
- 44: Signale der Kraftsensoren 42, 43

## Patentansprüche

1. Gerät (10)
mit einer Steuereinrichtung (22) zum Betrieb eines Instruments (12), einer Sonde, eines Endoskops, eines anderweitigen Endgeräts, einer Beatmungsvorrichtung oder einer sonstigen Baugruppe unter der Regie der Steuereinrichtung (22),
mit wenigstens einem ein berührungssensitiven Eingabeelement (15), das an die Steuereinrichtung (22) angeschlossen ist, um Nutzereingaben repräsentierende Signale (24) an die Steuereinrichtung (22) zu liefern,
mit einem Sperrmodul (25) zur wenigstens teilweisen Sperrung des Eingabeelements (15) gegen Eingaben.

2. Chirurgiegerät nach Anspruch 1, **dadurch gekennzeichnet, dass** das Sperrmodul (25) an oder in dem Eingabeelement (15), in der Steuereinrichtung (22) oder als gesondertes Modul (25) ausgebildet ist.

3. Chirurgiegerät nach Anspruch 1, **dadurch gekennzeichnet, dass** das Sperrmodul (25) lediglich eine einzige Nutzereingabe zum Entsperren des Eingabeelements (15) freigibt.

4. Chirurgiegerät nach Anspruch 3, **dadurch gekennzeichnet, dass** die Nutzereingabe durch eine charakteristische unter Berührung des Eingabeelements (15) auszuführende Geste gebildet ist.

5. Chirurgiegerät nach Anspruch 1, **dadurch gekennzeichnet, dass** der das Eingabeelement (15) Elektroden zur resistiven oder kapazitiven Berührungserfassung aufweist, die mit elektrischen Abfrageimpulsen(35) beaufschlagt sind, um eine Berührung zu erkennen, wobei das Sperrmodul (25) die Aussendung und/oder Auswertung der Abfrageimpulse (35) verlangsamt, um das Eingabeelement (15) nur auf lange Berührungen reagieren zu lassen, oder unterdrückt, um das Eingabeelement (15) zu sperren.

6. Chirurgiegerät nach Anspruch 1, **dadurch gekennzeichnet, dass** das Sperrmodul (25) einen Diskriminatorblock (33) zur Erfassung einer charakteristischen Signatur in dem von dem Eingabeelement (15) gelieferten Signal (23) aufweist.

7. Chirurgiegerät nach Anspruch 1, **dadurch gekennzeichnet, dass** zur Freigabe des Sperrmoduls (25) eine Entriegelungstaste (18) vorgesehen ist.

8. Chirurgiegerät nach Anspruch 1, **dadurch gekennzeichnet, dass** zur Freigabe des Sperrmoduls eine Krafterfassungseinrichtung (42, 43) vorgesehen ist, die mit dem Eingabeelement (15) verbunden ist.

9. Chirurgiegerät nach Anspruch 1, **dadurch gekennzeichnet, dass** zur Freigabe des Sperrmoduls (25) eine optische Erfassungseinrichtung (38) zur Erfassung der Präsenz einer Bedienperson vor dem Eingabeelement (15) vorgesehen ist.

10. Chirurgiegerät nach Anspruch 8 oder 9, **dadurch gekennzeichnet, dass** die Krafterfassungseinrichtung (42, 43) oder die optische Erfassungseinrichtung (38) zur Erzeugung eines die Berührungsposition kennzeichnenden Signals eingerichtet ist und dass eine Vergleichereinrichtung (36) vorgesehen ist, die eine gültige Eingabe signalisiert, wenn die von dem Eingabeelement (15) erfasste Position mit der von der Krafterfassungseinrichtung (42, 43) oder der optischen Erfassungseinrichtung (38) erkannten Position innerhalb einer Toleranz übereinstimmt.

11. Verfahren zur Vermeidung von durch elektromagnetische Störsignale verursachten Scheineingaben an einem Chirurgiegerät (10), das eine den Störsignalen ausgesetzte Eingabeeinrichtung (15) und eine Steuereinrichtung (22) aufweist, die von der Eingabeeinrichtung (15) Signale (23) erhält, die eine Nutzereingabe kennzeichnen, wobei sich unter den Signalen (23) Schein-Eingabesignale befinden können, die eine Nutzereingabe vortäuschen,
wobei zur Unterdrückung von Schein-Eingabesignalen nur solche Eingabesignale (23) als gültig zugelassen werden, die in vorgegebener Koinzidenz mit anderen, zur Eingabefreigabe dienenden Eingabesignalen stehen.

12. Verfahren zur Vermeidung von durch elektromagnetische Störsignale verursachten Scheineingaben an einem Chirurgiegerät (10), das eine den Störsignalen ausgesetzte Eingabeeinrichtung (15) und eine Steuereinrichtung (22) aufweist, die von der Eingabeeinrichtung (15) Eingabesignale (24) erhält, die eine Nutzereingabe kennzeichnen, wobei die Eingabeeinrichtung (15) nur dann aktiviert wird, wenn anderen Eingabesignalen vorliegen oder empfangen worden sind, die einer vorgegebenen Signatur entsprechen.

13. Verfahren nach Anspruch 11 oder 12, **dadurch gekennzeichnet, dass** ein Sperrmodul (25) vorgesehen ist, das Eingaben nach Nutzereingaben nach einer Periode der Nichtbenutzung der Eingabeeinrichtung (15) gegen weitere Eingaben sperrt.

14. Verfahren nach Anspruch 13, **dadurch gekennzeichnet, dass** das Sperrmodul (25) von dem Eingabeelement (15) gelieferte Signale (23) auswertet und nach Sperrung des Eingabeelements (15) Eingabesignale (24) erst dann einer weiteren Verarbeitung zuführt, wenn es in den Signalen (23) eine bestimmte Signatur erfasst hat.

15. Verfahren nach Anspruch 13, **dadurch gekennzeichnet, dass** das Sperrmodul (25) die Eingabesignale (24) erst dann einer weiteren Verarbeitung zuführt, wenn es ein Freigabesignal empfangen hat.
